# EUROPEAN PATENT APPLICATION

(11) **EP 1 760 616 A2**
(43) Date of publication of application: **07.03.2007**
(21) Application number: 06119125.0
(22) Date of filing: 17.08.2006
(51) Int. Cl.: G06F 19/00

(54) **Method and apparatus for collecting patient data for risk management**

(30) Priority: 19.08.2005 US 709429 P
(71) Applicant: Arellano, Felix, 03724 Moraira (ES)
(72) Inventor: Arellano, Felix, 03724 Moraira (ES)
(74) Representative: Carpintero Lopez, Francisco

(57) **Abstract**

A risk management method and associated tool is used to identify, assess, manage, and mitigate health risks associated with a broad range of medications, medical devices, procedures, and associated practices employed by practioners in the health field in the care and treatment of patients. The method may include identifying a parameter to study; searching a database to identify members of a population appropriate to the parameter; identifying a plurality of personnel responsible for prescribing an aspect of the parameter to the members of said population; and soliciting information from the personnel concerning risks associated with the aspect.

## Description

### BACKGROUND OF THE INVENTION

### RELATED APPLICATION

This application claims the benefit of the filing date of Provisional Application No. 60/709,429, filed August 19, 2005.

### FIELD OF THE INVENTION

The invention relates to identifying, assessing, managing and mitigating health risks associated with a broad range of medications, medical devices, procedures, and associated practices employed by practioners in the health field in the care and treatment of patients.

### DESCRIPTION OF THE RELATED ART

Risk Management is an iterative process of assessing the benefit-risk balance of a medical intervention and, as necessary, intervening to assure that the benefit-risk balance is optimized. This process requires ongoing surveillance for new events and assessment of the magnitude and impact of events that are already under consideration. Interventions to minimize identified risks and measurement of the success of those interventions are critical to the enduring efforts to maximize benefit and minimize risk Partially fueled by recent drug withdrawals there is increased pressure on regulatory agencies to demand better systems of risk management.

### SUMMARY OF THE INVENTION

According to one aspect of the invention, a method of risk management includes the steps of identifying a parameter to study; searching a database to identify members of a population appropriate to the parameter; identifying a plurality of personnel responsible for prescribing an aspect of the parameter to the members of the population; and soliciting information from the personnel concerning risks associated with the aspect.

According to a feature of the invention, the parameter to study may be selected from the group consisting of medications, medical procedures, medical devices, and treatment protocols.

According to another feature of the invention, the database may include populations of patients receiving, being treated according to, and or using one of the group. The database may further include linkages between the members of the population and the personnel

According to another feature of the invention, a step may be included to inform the personnel of a risky activity associated with prescribing the aspect of the parameter. The "aspect" may include, for example, a medication for which use is contraindicated for at least some members of the population.

According to another feature of the invention, steps of receiving responses may be included for collecting information and a step of generating may be included to generate an aggregated report from the responses.

According to another feature of the invention, the step of soliciting information from the personnel may include steps of contacting the personnel; posting questions soliciting the information on a website; and collecting responses to the questions from the website.

According to another feature of the invention, an evaluation step may be included for evaluating an effectiveness of the step of soliciting information from the personnel concerning risks associated with the aspect in affecting a behavior of the personnel in connection with the risk.

According to another aspect of the invention, a method includes the steps of identifying a medication associated with a potential risk; identifying a population of patients receiving the medication; identifying a plurality of practitioners having control of the patients receipt of the medication; and soliciting information from the practitioners about their behavior with respect to the medication,

According to another aspect of the invention, a risk management tool includes software stored in a machine readable media, the software including programming for performing the steps of identifying a parameter to study; searching a database to identify members of a population appropriate to the parameter; identifying a plurality of personnel responsible for prescribing an aspect of the parameter to the members of the population; and soliciting information from the personnel concerning risks associated with the aspect.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Figure 1 is a flow chart of a method implemented by an Information Gathering and Risk Characterization Tool for gathering information and characterizing a risk from prescribers indicating their awareness of and/or behavior with respect to a subject of medical scrutiny such as a medication, device, procedure, etc.;

Figure 2 is a flow chart of a method implemented by a Risk Minimization Tool for minimizing risk of users of a subject of medical scrutiny (such as a medication, device, procedure, etc.) by making them aware of applicable issues, potential dangerous behaviors, etc.;

Figure 3 is a flow chart of a method implemented by an Interventional Risk Minimization Tool for intervening when a prescribing behavior has been identified as dangerous or in need of modification;

Figure 4 is a diagram illustrating distinctions between the Information Gathering and Risk Characterization, Risk Minimization and Interventional Risk Minimization Tools;

Figure 5 is a flow chart of a method implemented by a Risk Management Evaluation Tool for measuring efficacy of interventional risk minimization actions;

Figure 6 is a flow chart of a method implemented by a Risk Management Evaluation Tool for identifying long-term effectiveness of a risk management program; and

Figure 7 is a screen shot of a sample questionnaire that might be provided to a prescriber to gather information about his/her prescribing procedures, make them aware of contraindications, and identify risky practices.

### DESCRIPTION OF THE PREFERRED EMBODIMENTS

The present invention includes embodiments directed to a physician-focused Risk Management (RM) process and supporting tools, software and associated apparatus implementing RM methods. While a preferred embodiment of the invention will be described based on use of The Health Improvement Network (THIN) database, embodiments and implementation of the invention may alternately use a variety of automated databases linking (i) information on prescribers, (ii) drugs, devices, indications, and/or other treatments and (iii) patients. For example, other existing databases include but are not limited to: GPRD (General Practitioners' Research Database), The VA (Veteran's Administration) database, Medicare, Medicaid, PharMetrics, Kaiser Permanente, United Healthcare, Pharmo, etc. Further, while embodiments of the invention are directed to use as an RM tool, a wide range of applications are possible including information gathering and collection over abroad range of requirements and fields including any question that has a root physician/patient with condition or prescription such as impact medical malpractice or patient's health insurance coverage has on patient treatment.

For ease of discussion, the following definitions of terms as used herein are provided:
Prescribing behavior: Actions taken by a health care provider relating to the treatment of a patient including, but not limited to the prescribing of a drug, employment and implementation of medical procedures and devices and recommending the use of medical devices.
Physician: Unless otherwise noted or apparent from context, broadly includes a person or entity subject to some form of monitoring such as, but not limited to, health care providers having direct responsibility for the treatment of patients such as physicians, nurses, pharmacologists, psychologists, medical staff, etc.
Questionnaire: all forms and methods in which information and responses may be solicited from a subject and or research about a subject may be conducted including, but not limited to, web-based forms, telephone interview, printed documents, etc. as well as the ability to communicate desired information to recipient using questionnaires as defined herein.
Risk Management (RM): an ongoing process of assessing the benefit-risk balance of a medical treatment and as necessary, intervening as well as communicating information to assure that the benefit-risk balance is optimized.
Parameter: the objective (risk, behavior, or communication) of the study.
Risk: exposure the change of injury or loss.
Population: in the context of mining a database for patients meeting the study parameter, population is the patients that need to be linked to the physician. Risky Behavior: a behavior by a prescriber (generally, but not necessarily, the prescription of a drug, employment and/or implementation of a medical procedure, etc.) that may have an elevated patient risk based on the presence of known, likely, suspected, potential or the like patient outcomes related to the treatment. Risky behavior in many cases results from a physician knowledge deficit Nonetheless, there are cases in which, in the professional judgment of the treating physician (even if he knew of the risk before initiating treatement or later became aware of the risk including through the use of the questionnaires described herein), the benefit of treatment outweighs the risks and the risky behavior will not be modified.
RMAP - Risk Minimization Action Plan: A plan aimed to minimize and identified risk (in this context associated with the use of medications). An RMAP can be self-initiated by a drug sponsor or in response to a mandate by a health authority,

According to a prevalent and common healthcare treatment paradigm:
Patient goes to physician
Physician recommends treatment e.g.
A prescribed medication (Rx) or combination of medications (prescriptions);
Medical device;
Patient education;
Other recommendations, e.g. exercise;
Risk communication;
Potential problem knowledge deficit by physician and patient understanding
Patient follows treatment

If there are side effects, adverse reactions, etc., then the patient tells his/her doctor and/or goes to hospital and reports of the incident may be voluntarily made to an appropriate regulatory agency depending on the infrastructure established and available in a particular country.

Within the global healthcare field Risk Management (RM) generally includes five major principals;
1. Risk measurement including estimation and evaluation of risk;
2. Determining an acceptable level of risk;
3. Risk management by intervention and control (the end result being a risk minimization action plan (RiskMAP));
4. Risk communication based on the interchange of information regarding risk; and
5. Risk management evaluation to measure the effectiveness of the risk minimization or risk control activities included in a RMAP.

For instance, while not the only RM guidelines; the FDA recently issued final guidelines on RM consistent with these principles.

According to a method of the invention, a physician-focused RM invention advances the field of healthcare (including, but not limited to, both pharmaceuticals and medical devices) RM for each of the five principles described above.

A feature of a method according to the invention is the ability to link information used for RM. Thus, a feature of a database supporting RM includes a capability to identify (through linking the information in a computer database) a prescriber in charge of a patient with the treatment e.g. prescriptions that the patient receives, procedures to which the patient is subject, condition patient has etc. Another feature is the ability to address the prescriber (such as a General Practitioner or "GP") with a series of questionnaires that can be customized depending on the type of tools used. The questionnaires are used to communicate information such as to inform the practitioner of known or potential risks in general and specific to their activities and behaviors, potentially or actually mitigate undesirable behaviors and intervene to avoid dangerous activities as well as elicit desired information.

Embodiments of the invention include a number of methods that may be implemented as "tools" to effectuate an RM program Certain of these tools may be implemented by a combination of software for storing, searching, mining and integrating information in, and from, one or more databases. The tools may be supported by various hardware systems such as personal computers, network-based servers, and similar devices. It should also be understood that these tools may be used individually or collectively as part of a RM approach or Risk Minimization Action Plan ("RMAP"). These tools include a/an:
Information gathering and/or risk characterization/evaluation tool;
Risk minimization tool;
Interventional risk minimization tool;
Risk management evaluation tool; and
Randomized risk management evaluation tool.

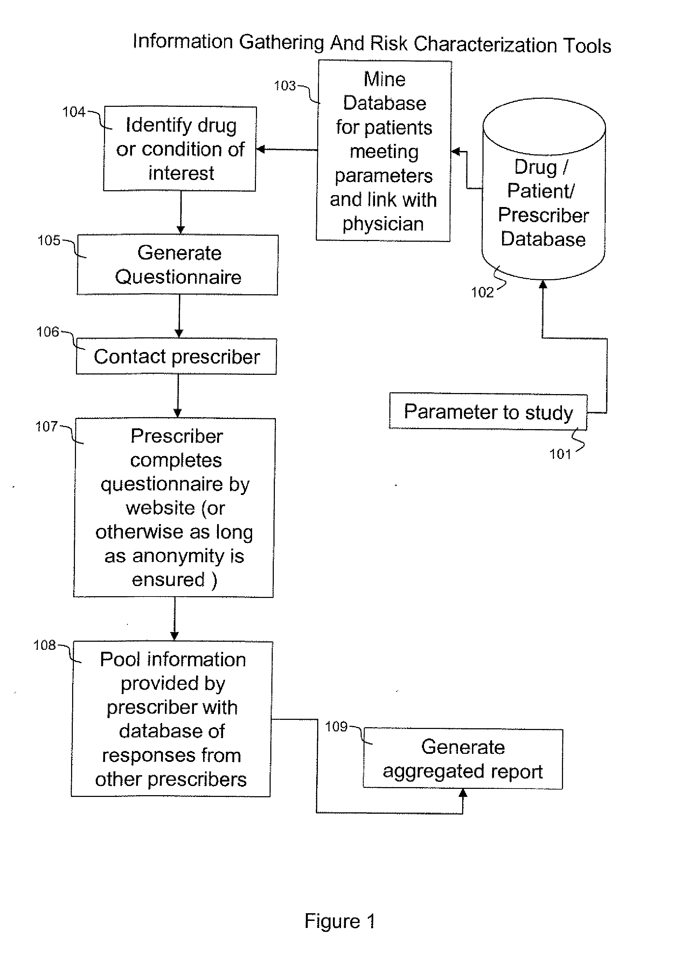

Figure 1 depicts a method implemented by an information gathering and/or risk characterization/evaluation tool according to an embodiment of the invention. This tool may be used to gather specific information from a physician and/or characterize, or put into context, a particular risk or a particular physician behavior or awareness with, or without, limitations related to specific patient groups, age, gender, conditions, or other limitations. The tool addresses information gathering regarding behaviors as well as identification and/or understanding of a particular risk. The risk may be dependent on factors not available from the database so that the existence of the risk and level of risk may need to be determined based on, for example, responses to the questionnaire.

A first step 101 is the identification of some "parameter", i.e., a subject risk, an appreciation for the current exposure or risk, or a behavior. For example, knowing that certain medications are not approved for use in certain populations or are otherwise contraindicated, we may want to know how many children under the age of 2 have been prescribed Elidel within the past 12 months. As these tools may be used independently or together, once a risk has been characterized this information can be used for RM including all forms of interventional RM described herein. For example, should the FDA at some time indicate that no more than 1% use of Elidel in the less than 2 years-old population is an acceptable risk, this 1% value may be adopted as an appropriate risk threshold.

Awareness/understanding/evaluation of a safety issue/risk may include such factors as:
an unapproved use;
contraindication or drug interaction;
side effects of a drug known by prescriber;
drug interactions;
other risks that are not contraindicated etc e.g., risk identified in literature but not on label; or
reasons for prescribing (e.g., aware of risk and benefit was greater than risk)
Awareness/understanding/evaluation of a safety issue/risk may be tailored to measure different levels of awareness or understanding including, for example, scientific (e.g. frequency) and psychological factors e.g., subjective perspective of risk i.e., Thalidomide use in men or failure to prescribe aspirin in stroke patients because of concerns of G/I bleeding
(Typically, risks associated with stroke are typically far more life threatening than risk of GI bleeding.)

Behavior that is the target of information gathering, monitoring, evaluation, warning, modification, interdiction, and/or other remedial action may include, for example:
Prescribing habits of a physician(s):
   In general;
   Preferred treatment for a particular disease and/or condition;
   Interaction between medications, e.g. when combining multiple drugs; and
   Various conditions that impact selection, e.g., other medications, conditions, specific patient or group requirements and concerns, etc.

Behavior may further include reasons why a physician elects, or is inclined to, recommend/prescribe or not recommend/prescribe a course of treatment such as a particular medication regime. For example, we may want to determine why or what factors, if any, could cause a physician to not place a patient on Warfarin after a stroke when Warfarin is the standard of care for most stroke patients.

Understanding, analyzing, and evaluating risks associated with behavior may further include gathering other information directly or indirectly affecting physicians' behavior (including prescribing and monitoring), such as:
health insurance;
malpractice concerns;
sources of product information (e.g., Sales representative v refereed Medical journals); and
physician understanding of risk, based on both "Intellectual" and "Psychological" factors, the latter being influenced by such diverse issues as, for example, malpractice concerns, the lay press; patient perception, etc.

Referring to Figure 1, the information sought is identified so that the database can be searched. The target information may be, for example, data concerning use of a medication to identify usage within a particular population. This process can be performed with a database 102 that has the capacity to link both the use of a drug or device with a prescriber and include information to identify and/or contact this prescriber (e.g., the previously referenced THIN). Using record linkage the database 102 can then be "mined" (e.g., searched) in step 103 to extract the desired information

For example, in the case of the THIN database, information is obtained from patient records downloaded directly from participating general practices. Patients are allocated a unique identification number, name and address are not collected. Variables collected include: practice and patient registration details, demographics, including age and sex of patient; medical diagnosis; all prescriptions, including repeats; events leading to withdrawal of a drug or treatment; referrals to specialists and hospitals; treatment outcomes including hospital discharge reports; smoking status, height, weight, immunizations, lab results, Variables included in the THIN database include patient identifier; administrative information, condition, intervention, short term outcome, major known confounders, and long term outcome. This information can be linked to the associated provider/prescriber identification in support of embodiments of the present invention.

Another suitable source of this information is the General Practice research Database (GPRD). The GPRD stores data about medical diagnosis, including comments; all prescriptions; events leading to withdrawal of a drug or treatment; referrals to hospitals; treatment outcomes, including hospital discharge reports where patients are referred to hospital; and miscellaneous patient care information e.g., smoking status, height, weight, immunizations, and lab results. Again, appropriate linkages to prescribing practitioners are added to provide prescriber information.

After database 102 is mined for patients associated with the parameter under study, such as drug and/or condition information (step 103) associated with the parameter under study, a resultant "population," i.e., drug or condition of interest, if any, e.g., diagnosis, age gender, other risk factors such as blood pressure and concomitant conditions, may be further identified at step 104 A suitable questionnaire can then be developed at step 105 to elicit additional information about the use of the subject medication, procedure, etc. The physician or other prescriber can then be contacted at step 106 using various methods and media, such as telephone, mail, e-mail, a suitable website, etc, Using such a vehicle, the physician receives and is asked to complete the questionnaire at step 107 According to a preferred embodiment, the questionnaire may be implemented as a directed series of questions presented on a secure website accessible over, for example, the Internet Responses to the questions are then collected and the information from multiple respondents pooled at step 108 to create, at step 109, a summary report which may or may not be devoid of specific physician identifiable information and may or may not be devoid of patient identifiable information so as to address privacy concerns and laws concerning anonymity. Thus, collecting and pooling the information from the response forms received from all prescribers supports generation of an aggregate report. Information on the prescribers' awareness and/or behavior may then be used for risk communication including but not limited to Risk Minimization or Interventional Risk Minimization Tools described herein.

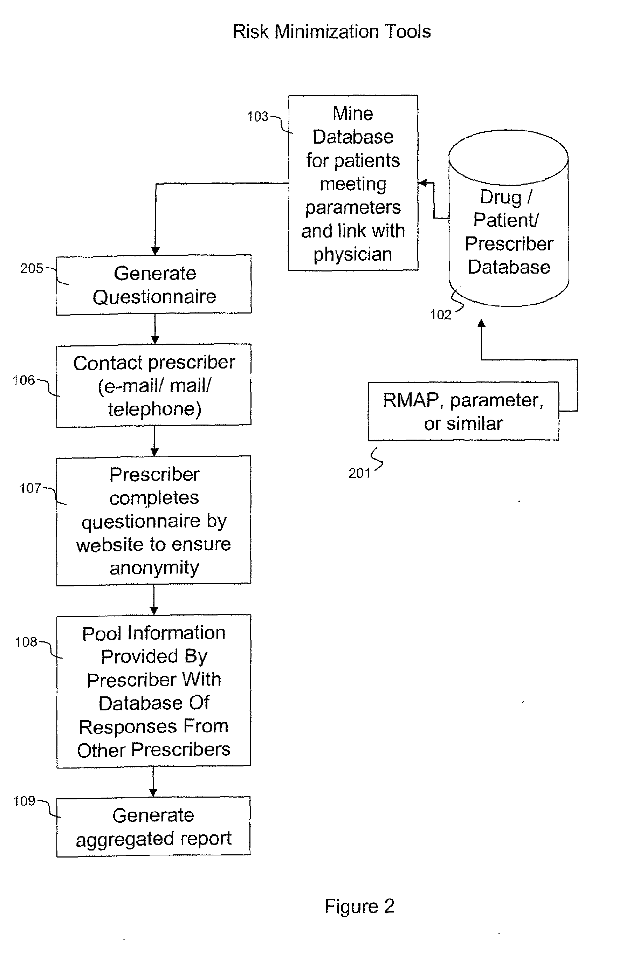

A method implemented by a risk minimization tool according to an embodiment of the invention is shown in the flow chart of Figure 2. This tool is used when there is an identified risk in the use of a particular drug or device (whether measured/quantified or not and whether or not subject to a risk management or minimization plan). Thus, the risk at stake needs to be known (e.g. the presence of a contraindication for prescribing a drug; not approved for particular treatments; or identified in a study but not yet published) although not necessarily quantified (e.g. it may not be known if it is something that would affect all patients with the condition that receive the drug or only some), In general this risk, since it is known, may be, but is not necessarily, documented in a RMAP.

Referring to Figure 2, a first series of steps identify all prescribers who use the particular drug or device with the identified risk. Thus, parameters defining the risk to be addressed are defined at step 201 and used to access database 102 resulting, at step 103, in information identifying a population of patients and the corresponding physicians prescribing the drug or procedure. At step 205 an appropriate questionnaire is formulated to communicate appropriate information and solicit additional information and apprise or remind the physician about the potentially risky behavior. That is, since an objective of the risk minimization tool is to minimize risk, the questionnaire sent to the prescriber will be targeted as such, that is, to physicians or other prescribers who are identified as using a particular medication or procedure under some condition that may entail an elevated risk whether or not there is predetermined threshold level. The risky behavior may be dependent on factors not available from the database so that the existence of the risk and level of risk may need to be determined based on, for example, responses to the questionnaire. As may be the case for all tools, risky behavior may not be modified because of physician professional judgement that the benefits outweigh the risks. The questionnaire may include, for example:
A reminder of the presence of a risk (e.g., a reminder that the use of another drug is contraindicated with the study drug); and
A reminder of the need to take specific measures before or during drug treatment (e.g. monitor liver function tests when using a drug, ensuring that a patients is not pregnant or is on proper contraception and/or is not within some other population for which there is an specific or identified elevated risk, etc)

While the questionnaire created at step 205 in this present example may have a substantial purpose of making the prescriber aware of a particular issue, it may also contain a set of questions for eliciting further information and data gathering (e.g., whether the physician was aware of the existence of the contraindication or not, what was the source for this information, what was the best way s/he got the information, etc).

Once the questionnaire is formulated, the physician may be contacted at step 106, again using various modes and media such as telephone, mail, e-mail, etc. Upon receipt, the physician completes the questionnaire at step 107 by responding appropriately. Responses may also be generated automatically by interfacing with data systems at the physician's practice. To avoid improper disclosure of personal information, an appropriate protocol may be used to implement and/or effectuate a firewall between an information soliciting or data collection system and the physician maintained patient records and data. Alternatively or in addition, as described above, a preferred embodiment may include having the physician go to a secure website so as to promote ease of responding to various questions of the questionnaire while maintaining/enhancing physician and/or patient anonymity. Responses supplied by the physician may be disassociated from information identifying a particular physician and assigned a reference code that may later be used to confirm or verify that a report was submitted. Such a verification might be useful to encourage physician participation if, for example, some limited form of immunity might be provided for reported actions that might otherwise provide liability for the practitioner.

The physician may or may not receive an honorarium for his participation for this tool or any of the tools contained herein.

Information from responses received from all prescribers is then collected and pooled (step 108) and an aggregate report is generated (step 109). Eventually, the information may be fed back to the client in a report and could be used as a basis for any risk communication or any other RM tool including but not limited to those tools described herein deemed desirable or necessary in view of the reported aggregate and/or individual behavior(s)

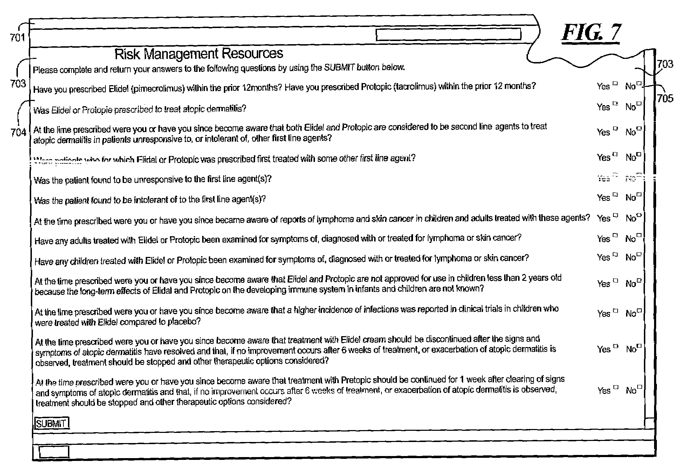

An example of use of the risk minimization tool in connection with a medication in which use for a certain population is not approved is given in connection with Figure 2 For example, ELIDEL (pimecrolimus) is a steroid-free cream prescribed for patients having mild or moderate eczema (atopic dermatitis). However, due to the unknown effects of Elidel on the developing immune system in infants and children, its use in children less than 2 years old is not approved by the FDA. Thus, using the risk minimization tool, appropriate parameters used to search database 102 may include the prescription of Elidel to patients under 2 years of age.
These parameters of the study result in identification in the database of all patients under 2 years of age receiving Elidel and, when properly linked, identification of those physicians prescribing Elidel to members of that patient population (step 103). An appropriate questionnaire is then formulated (step 205). An example of a questionnaire is presented in Figure 7 depicting a web browser 701 displaying a web page 703 including the questionnaire. Although the sample questionnaire includes questions 704 eliciting yes/no responses 705, other forms or responses may be employed including, for example, multiple choice selections, drop-down menus, free-form text entry, etc. Further, although a fixed set of questions are shown, the questionnaire may be dynamic so that appropriate questions are supplied and/or inapplicable questions skipped based on previous answers.

Note that, in the example of Figure 7, the questionnaire not only solicits information to be used in assessing the overall risk (eg, how many physicians indicate that they are prescribing a medication contrary to established protocol, etc.), but directs the physician's attention to, or informs, the physician of the potentially risky behavior.

Having identified the prescribers to receive the questionnaire or survey, at step 106 they are contacted by an appropriate means (e.g., mail, e-mail, etc.) and supplied with or told how to obtain the questionnaire (e.g., visit and/or link to a website). Upon completion at step 107, survey or questionnaire results received from the targeted group of prescribers are then pooled at step 108 and used to generate an appropriate aggregate report (step 109). Such an aggregate report might include, for example,
(i) the number of physicians prescribing Elidel to the target population of patients under 2 years old,
(ii) the percentage of those physicians prescribing to that population who were unaware of the appropriate protocol and/or contraindications with respect to the targeted patient population,
(iii) the percentage of those physicians prescribing that were aware of the unapproved use but prescribed Elidel because there were no suitable alternatives identified, and (iv) the percentage of those physicians identifying conditions under which use of Elidel was appropriate in spite of known or possibly inapplicable contraindication

Another example of the use of the risk minimization tool in connection with a medication in which the use for a certain population is contraindicated at the time of drug approval or at some point post approval is given in Figure 2. For instance, Bextra (valdecoxib), prior to the withdrawal from the market, had a label change related to increased risk of blood clotting especially in patients who have just had Coronary Artery Bypass Graft surgery (CABG). Similar to the approach described above (e.g., identify physicians prescribing Bextra to CABG patients and formulate appropriate questionnaire), the study may seek to communicate the new risk and solicit information.

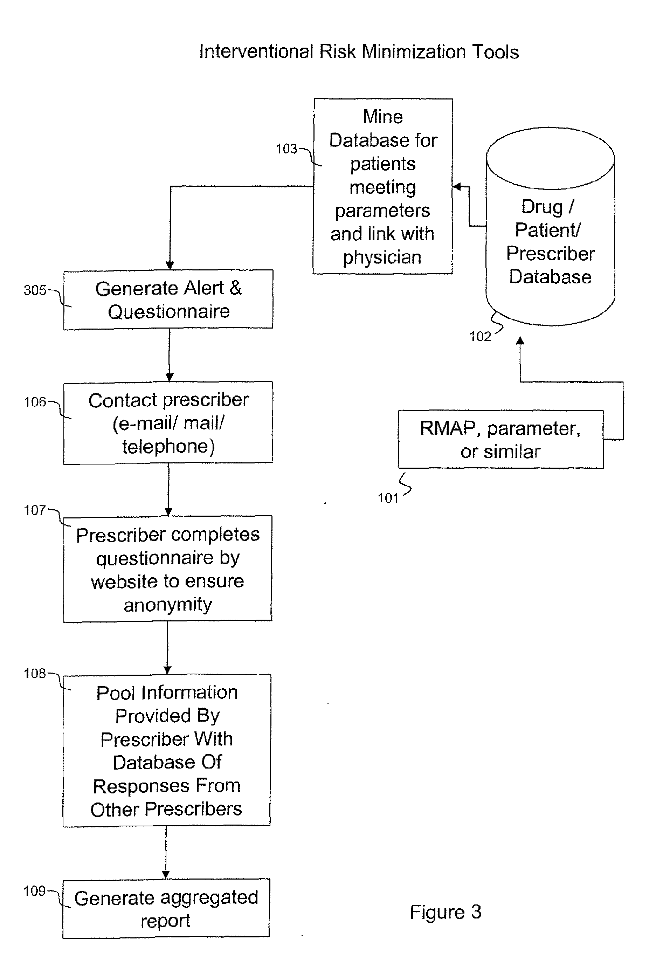

An interventional risk minimization tool method is depicted in Figure 3. In this example, rather than merely collecting information or identifying a potential risk, an actual risky behavior and/or hazardous condition has been identified and appropriate action, is advised. Thus, this tool is directed to intervening once a prescribing behavior is determined to merit some form of modification. A difference between the risk minimization tool and interventional risk minimization tool is that the latter requires that, in addition to a risk being known, a risky behavior by a particular prescriber has also be identified. That is, it is not sufficient to know that a given risk exists but it must be known that a prescriber has done something that may subject a patient to this risk. Thus, the prescriber will be notified that he/she has prescribed a drug or a device in a setting, or under conditions, that is/are considered the subject of an intervention.

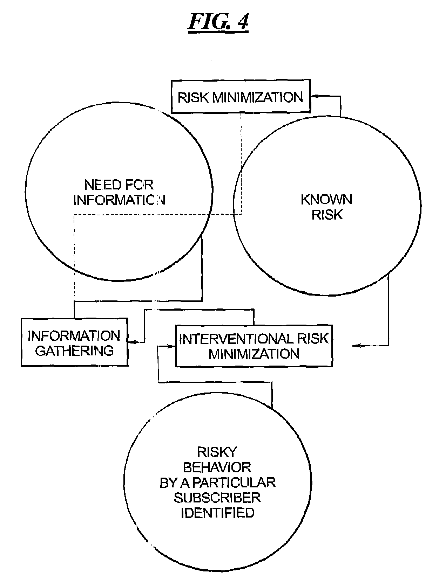

For example, a prescriber may be told that he/she has prescribed a patient a drug that is contraindicated or may interact with another drug. As with the risk minimization tool, the questionnaire may have a communication aspect or warning component but could also be used for information gathering purposes. For example, the questionnaire may ask whether the prescriber was aware of the risk (e.g., knew of the existence of contraindication or potential for interaction at the time the medication was prescribed and/or at some subsequent time such as the case of a physician prescribing Accutane to a woman of childbearing years without confirming that she has suitable birth control), whether he/she has modified or intends to modify the prescription due to the intervention, what is the best method of communication, etc. The different emphasis of the three tools is depicted in Figure 4.

Referring back to Figure 3, a method employed and/or implemented by a interventional risk minimization tool is shown. Similar to the prior methods and tools, some initial steps 101-103 result in identification of all prescribers who engage in some behavior identified as risky (i.e., "risky behavior") An appropriate questionnaire is constructed at step 305. Since an objective of this tool is to minimize risk by changing the risky behavior of the physician, the questionnaire sent to the prescriber will be targeted as such. This may include:
Notice that the prescriber has prescribed a drug or device in a manner that may place the patient receiving the drug at risk for a known side effect or adverse reaction; and
The need to take specific measures before or during drug treatment (e.g. monitor liver function tests when using a drug, ensuring that a patients is not pregnant or is on proper contraception, etc).

While the questionnaire in this case will have one object of apprising the prescriber of and emphasizing a particular issue, it may also contain a set of questions for further information gathering (e.g., whether he changed the treatment and why, whether the physician was aware of the existence of the contraindication or not, what was the information source for this information, what was the best / most effective way he/she got the information, etc).

Step 106 includes contacting the physician via, for example, telephone, mail, e-mail, etc. The Physician completes and returns the questionnaire at step 107. As per prior described preferred embodiments, questionnaire completion may be supported by a suitable website to facilitate collection of information and address privacy concerns. Responses to the questionnaires are collected and the information pooled at step 108 and an aggregate and/or summary report is generated at step 109. As before, the information gathered including the aggregated summary information may be fed back to the client in a summarized form and may be used as a basis for any desirable or required risk communication. As is the case with all of the tools described herein, the client may be, for example, a pharmaceutical company, an insurance carrier or group, a regulatory body, professional organization, an association or organization associated with the participating physicians, hospitals, employers, etc.

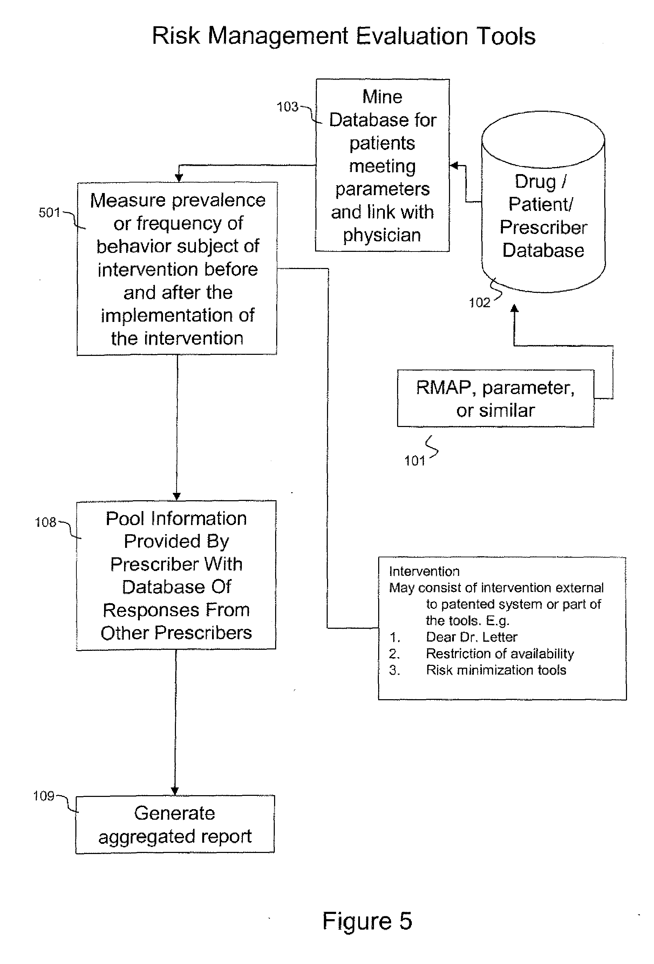

In addition to collecting risk-related information, identifying, managing, mitigating, and intervening to control risk, embodiments of the invention envision methods and tools for collecting data for evaluating the effectiveness of these RM processes and tools. Thus, two risk management evaluation tools implement the methods and steps depicted in Figures 5-6. These tools measure the effectiveness of the tools described above or of the implementation of a Risk Minimization Action Plan for a particular product. The first evaluation tool compares prescribing statistics or other activities over some period of time before and after implementation of the RM tools, while the second evaluation tool compares a test group with which the RM tools are used with a control group.

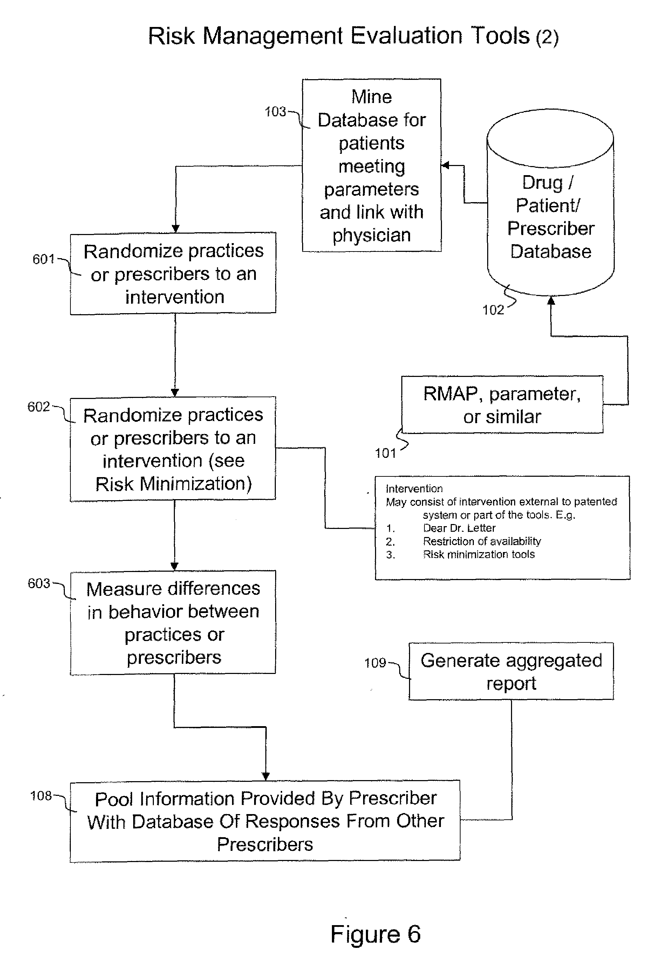

The two evaluation tools include the following:
The method and corresponding tool depicted in Figure 5 is directed to situations wherein there is an established risk for which an interventional task is described and an appropriate parameter (e.g., the prevalence of a contraindicated prescription, the incidence rate of a particular side effect, etc) can be measured before and after the intervention.
The method and corresponding tool depicted in Figure 6 is directed to a more generalized and anonymous evaluation method including randomly assigning some practices (and all of its prescribers) to be the subject of an intervention (e.g., the submission of a questionnaire) and not those of a "control" group. The effectiveness of the intervention is then measured and compared between the two groups (e.g., the test and control groups).

Referring to Figure 5, according to the first mentioned RM Evaluation method, an initial series of steps implement a historical investigation into the prevalence or frequency of the condition subject of the risk intervention (e.g. how frequent was the use of a contraindicated drug before the intervention was launched) Thus, parameters defining the risk to be addressed are defined at step 101 and used to access database 102 resulting, at step 103, in information identifying a group of physicians prescribing the drug or procedure that is the subject of the RMAP. To do this the database may be mined through appropriate record linkages and the outcome of interest can be measured. The same process may be repeated using a time frame after the intervention implementation (e.g., how frequent was the use of a contraindicated after applying the intervention tool). The pre and post-intervention results are compared at step 501 to provide a metric of the effectiveness of the intervention. In general the threshold to define effectiveness of the RM should be predefined (e.g., a RMAP will be considered effective if it reduces the use of the contraindicated drug by 50% after three months of implementing the risk minimization tool). This information is pooled at step 108 to provide summary information and statistics devoid of individual and/or practice identification and/or specifics and an aggregated report generated at step 109.

The second type of RM evaluation combines the use of the risk minimization tool and evaluation over time. Using this system practices are randomized into two groups, one receiving the risk minimization tool, the other acting as a control group, and the resultant differences in resultant behavior measured. For example, some practices may receive the risk minimization tool and some will not and the frequency of use of the contraindicated drug before and after the implementation will be measured. This method has more scientific validity but may only be used in cases where it will be considered ethical to do so. For example if a risk minimization strategy is already been proven to work or the condition to be prevented is life-threatening, this may not be used Thus, with reference to Figure 6, steps 101-103 result in identification of practices employing some practice that is considered risky. At steps 641 and 602 the identified practices are partitioned into two groups, one that will be part of an intervention process, the other acting as a control group. At step 603 the differences in behavior are measures, the information pooled at step 108 and an aggregated report generated at step 109.

The evaluation method include collecting and pooling information and then generating an aggregate or summary report Eventually, the information may be fed back to the client in a summarized form and may be used for as a basis for needed risk communication.

According to an aspect of the invention, the prescriber is the center of the process because the effectiveness of a risk management tool will only be as good as the information that the prescriber receives and uses. These tools will only be effective as long as the prescribers are willing to collaborate, see the tools as useful, and do not see it as a threat. It will be of paramount importance to ensure complete anonimization of both the prescriber and the patient. The prescriber will need to be reassured that this will be not a tool to evaluate their prescribing habits, or that the data that they will contribute to the system will be used in any other way than that intended. For example, it may not be available in case of litigation. Finally, the physician will reed to be compensated appropriately.

In this disclosure there is shown and described only the preferred embodiments of the invention and but a few examples of its versatility. It is to be understood that the invention is capable of use in various other combinations and environments and is capable of changes or modifications within the scope of the inventive concept as expressed herein. Furthermore, it should be noted and understood that all publications, patents and patent applications mentioned in this specification are indicative of the level of skill in the art to which the invention pertains. All publications, patents and patent applications are herein incorporated by reference to the same extent as if each individual publication, patent or patent application was specifically and individually indicated to be incorporated by reference in its entirety.

## Claims

1. A method of risk management comprising the steps of identifying a parameter to study;
searching a database to identify members of a population appropriate to said parameter; identifying a plurality of personnel responsible for prescribing an aspect of said parameter to said members of said population; and
soliciting information from said personnel concerning risks associated with said aspect.

2. The method according to claim 1 wherein said parameter to study is selected from the group consisting of medications, medical procedures, medical devices, and treatment protocols.

3. The method according to claim 1 wherein said database includes populations of patients receiving, being treated according to, and or using one of said group.

4. The method according to claim 1 wherein said database includes linkages between said members of said population and said personnel.

5. The method according to claim 1 including a step of informing said personnel of a risky activity associated with said prescribing said aspect of said parameter.

6. The method according to claim 1 wherein said aspect of said parameter includes a medication for which use is contraindicated for at least some members of said population.

7. The method according to claim 1 further comprising the steps of receiving responses to said step of soliciting and generating an aggregated report from said responses.

8. The method according to claim 1 wherein said step of soliciting information from said personnel includes the steps:
of contacting said personnel;
posting questions soliciting said information on a website; and
collecting responses to said questions from said website.

9. The method according to claim 1 further comprising a step of evaluating an effectiveness of said step of soliciting information from said personnel concerning risks associated with said aspect in affecting a behavior of said personnel in connection with said risk.

10. A method comprising the steps of:
identifying a medication associated with a potential risk;
identifying a population of patients receiving said medication;
identifying a plurality of practitioners having control of said patients receipt of said medication; and
soliciting information from said practitioners about their behavior with respect to said medication.

11. The method according to claim 10 further comprising a step of notifying said practitioners about said potential risk.

12. The method according to claim 11 further comprising a step of evaluating an affect of said step of notifying said practitioners in modifying a behavior of said practitioners.

13. The method according to claim 10 further comprising a steps of collecting responses from said practitioners and generating a summary report based on said responses.

14. The method according to claim 10 wherein said control of said patients receipt of said medication includes a step of prescribing said medication to said patients.

15. A risk management tool comprising a software stored in a machine readable media, said software including programming for performing the steps of:identifying a parameter to study;
searching a database to identify members of a population appropriate to said parameter;
identifying a plurality of personnel responsible for prescribing an aspect of said parameter to said members of said population; and
soliciting information from said personnel concerning risks associated with said aspect,

16. The risk management tool according to claim 15 wherein said parameter to study is selected from the group consisting of medications, medical procedures, medical devices, and treatment protocols.

17. The risk management tool according to claim 15 wherein said database includes populations of patients receiving, being treated according to, and or using one of said group.

18. The risk management tool according to claim 15 wherein said programming further performs a step of informing said personnel of a risky activity associated with said prescribing said aspect of said parameter.

19. The risk management tool according to claim 18 wherein said programming further performs a step of evaluating an effectiveness of said step of informaint said personnel in changing a behavior of said personnel in connection with said risky activity.

20. The risk management tool according to claim 15 wherein said programming further performs a step of generating a webpage presenting a series of questions and responses for soliciting and collecting said information from said personnel concerning said risks associated with said aspect
